# EUROPEAN PATENT APPLICATION

(11) **EP 3 878 518 A1**
(43) Date of publication of application: **15.09.2021**
(21) Application number: 19901334.3
(22) Date of filing: 18.12.2019
(51) Int. Cl.: A61Q 11/00, A61Q 13/00, A23L 27/00, A61K 8/33, A23L 2/56

(54) **FLAVOR COMPOSITION**

(30) Priority: 21.12.2018 JP 2018239524; 22.08.2019 JP 2019152072
(71) Applicant: Takasago International Corporation, Tokyo 144-8721 (JP)
(72) Inventor: MURATA, Ariaki, Hiratsuka-shi, Kanagawa 254-0073 (JP); ZAIZEN, Kyoko, Hiratsuka-shi, Kanagawa 254-0073 (JP); TAKAHASHI, Tomoya, Hiratsuka-shi, Kanagawa 254-0073 (JP); KURABE, Aki, Hiratsuka-shi, Kanagawa 254-0073 (JP); TAIRA, Kazuya, Hiratsuka-shi, Kanagawa 254-0073 (JP)
(74) Representative: Hoffmann Eitle
(86) International application number: PCT/JP2019/049716
(87) International publication number: WO 2020/130059

(57) **Abstract**

The present invention addresses the problem of providing a flavor composition capable of imparting or enhancing excellent flavor characteristics. The present invention relates to a flavor composition containing 8-decenal, and preferably, to a flavor composition for being added to at least one product selected from the group consisting of foods and drinks, oral compositions, and tobacco products.

## Description

### Technical Field

The present invention relates to a flavor composition.

### Background Art

In order to add flavors to foods or enhance flavors of foods, for example, essential oils obtained from natural products are sometimes used. There are also common methods to add or enhance flavors by adding various compounds instead of essential oils.

8-Decenal, the compound used in the present invention, is described in Patent Literature 1 as one of active aldehydes released from pro-fragrances. Also, in Patent Literature 2, a mixture of fragrances containing 6-decenal, 7-decenal, and 8-decenal in a specific ratio is described. Furthermore, in Non-Patent Literature 1, 8-decenal is described as a component contained in the essential oil of Gentiana veitchiorum belonging to Gentianaceae.

### Citation List

### Patent Literature

Patent Literature 1: US 8,557,262 B2
Patent Literature 2: US 8,461,100 B1

### Non-Patent Literature

Non-Patent Literature 1: ShiZhen Guoyi Guoyao (2009), 20(2), p347-348

### Summary of Invention

### Technical Problem

However, any flavor compositions containing 8-decenal are not described in Patent Literature 1, Patent Literature 2 and Non-Patent Literature 1.

In addition, Patent Literature 1, Patent Literature 2, and Non-Patent Literature 1 do not describe that 8-decenal is used in products such as foods or beverages, oral compositions, or tobacco products.

An object of the present invention is to provide a flavor composition capable of imparting or enhancing excellent flavor characteristics (for example, at least one selected from a peel feeling, a juice feeling, a ripening feeling, a volume feeling, a grain feeling and a tea leaf feeling, etc.). Another object of the present invention is to provide a product in which excellent flavor characteristics have been imparted or enhanced using the flavor composition, and a method for producing the product.

### Solution to Problem

As a result of intensive study to achieve the above objects, the present inventors found that 8-decenal, which has never been used as a flavor ingredient, can be used to impart or enhance excellent flavor characteristics, leading to the completion of the present invention.

That is, the present invention encompasses the following subject matters [1] to [7].
[1] A flavor composition comprising 8-decenal.
[2] The flavor composition according to [1], wherein 8-decenal is (8Z)-decenal.
[3] The flavor composition according to [1] or [2], which is for addition to at least one product selected from the group consisting of foods or beverages, oral compositions, and tobacco products.
[4] The flavor composition according to [3], wherein the product has a citrus-like flavor.
[5] The flavor composition according to [4], wherein the citrus is at least one selected from the group consisting of grapefruit, orange, lemon, lime, yuzu, blood orange, bitter orange, tangerine, mandarin orange, satsuma mandarin orange, amanatsu, natsumikan, hassaku, pomelo, kabosu, sudachi, hebesu, bergamot, and sweetie (oroblanco).
[6] A food or beverage, oral composition or tobacco product, comprising the flavor composition according to any one of [1] to [5].
[7] A method for producing a food or beverage, oral composition, or tobacco product, the method comprising adding the flavor composition according to any one of [1] to [5].

### Advantageous Effects of Invention

By adding the flavor composition in the present invention to at least one product selected from the group consisting of, for example, foods or beverages, oral compositions, and tobacco products, excellent flavor characteristics can be imparted or enhanced.

### Description of Embodiments

The present invention is described in detail below. In the present description, "mass" is synonymous with "weight".

The flavor composition in the present invention contains 8-decenal, and is preferably used for being added to at least one product selected from the group consisting of foods or beverages, oral compositions, and tobacco products (hereinafter referred to as "specific products" in some cases).

Because of 8-decenal contained in the flavor composition in the present invention, excellent flavor characteristics can be imparted to the specific products or excellent flavor characteristics of the specific products can be enhanced.

8-Decenal can be synthesized, for example, by the method described in US 8,461,100 B1.

As for 8-decenal, there are cis and trans isomers, but any isomer or isomer mixture can be used in the present invention. Among these, isomers and isomer mixtures with a high ratio of (8Z)-decenal are preferred. The ratio of (8Z)-decenal [mass of (8Z)-decenal/mass of (8E)-decenal] is preferably 100/0 to 60/40, and is more preferably 100/0 to 80/20. As for 8-decenal, it is most preferable to use only (8Z)-decenal.

In addition, in order to impart or enhance superior flavor characteristics by the flavor composition in the present invention, it is preferable that the specific product has a citrus-like flavor.

As the citrus, examples thereof include grapefruit, orange, lemon, lime, yuzu, blood orange, bitter orange, tangerine, mandarin orange, satsuma mandarin orange, amanatsu, natsumikan, hassaku, pomelo, kabosu, sudachi, hebesu, bergamot, and sweetie (oroblanco), but the citrus is not limited to these.

The content of 8-decenal in the flavor composition in the present invention may be in the range of, for example, 0.00001 ppm to 10,000 ppm, preferably 0.0001 ppm to 1,000 ppm, and more preferably 0.001 ppm to 100 ppm, based on the mass of the flavor composition. In this description, "ppm" means "0.0001 mass%".

The content of 8-decenal in the specific product may be in the range of, for example, 0.00001 ppb to 10,000 ppb, preferably 0.0001 ppb to 1,000 ppb, and more preferably 0.001 ppb to 100 ppb, based on the mass of the specific product. In this description, "ppb" means "0.0000001 mass%".

The flavor compositions in the present invention may contain, as necessary, commonly used solvents such as water or ethanol; or fixatives such as ethylene glycol, propylene glycol, dipropylene glycol, hexylene glycol, glycerin, triethyl citrate, medium-chain fatty acid triglycerides, or medium-chain fatty acid diglycerides.

As for the flavor composition in the present invention, in addition to the above-described ingredients, the other flavor ingredients may be mixed therewith to impart excellent flavor characteristics to the specific products or enhance excellent flavor characteristics of the specific products.

As the other flavor ingredients that may be mixed, various synthetic flavor ingredients, natural flavor ingredients, natural essential oils, plant extracts, etc. may be mentioned, and for example, examples thereof include synthetic flavor ingredients, natural flavor ingredients, natural essential oils, etc. as described in "Japan Patent Office Gazette, Collection of Well-Known and Conventional Techniques (Flavoring) Part II: Flavoring Ingredients for Food Products, p.88 to 131, published on January 14, 2000.

The food or beverage, oral composition, or tobacco product in the present invention is obtained by adding the flavor composition in the present invention to the specific product.

As the method of adding the flavor composition in the present invention, examples thereof includes, for example, a method of dropping the flavor composition in the present invention into the specific product, a method of mixing the flavor composition in the present invention with the specific product and stirring the mixture, and the like.

As the food or beverage, examples thereof include, for example, beverages such as carbonated beverages, soft drinks, fruit juice drinks, milk beverages, lactic acid bacteria beverages, energy drinks, soy milk, and tea drinks; alcoholic drinks such as shochu mixed with soda water, cocktail drinks, sparkling liquor, fruit wine, and alcoholic drinks for medical purposes; desserts such as ice cream, ice milk, lacto-ice, sherbet, yogurt, pudding, jelly, and daily dessert; confectioneries such as caramel, candy, tablet, cracker, biscuit, cookie, pie, chocolate, snack, and chewing gum; soups such as Japanese soup, and Western soup; jams; flavor seasonings; various instant beverages; and various instant foods. Among these, beverages or alcoholic drinks are preferred.

The amount of the flavor composition in the present invention added to a food or beverage depends on the kind or form of the product, and may be preferably 0.001 mass% to 10 mass%, and more preferably 0.01 mass% to 5 mass%, based on the mass of the food or beverage to which the flavor composition has not been added.

As the oral compositions, examples thereof include, for example, toothpastes, oral rinses, mouthwashes, troches, chewing gums, and the like.

The amount of the flavor composition in the present invention added to the oral composition depends on the kind or form of the product, and may be preferably 0.01 mass% to 5 mass%, and more preferably 0.1 mass% to 3 mass%, based on the total mass of the oral composition.

As the tobacco products, examples thereof include, for example, cigarettes, electronic cigarettes, and the like.

The amount of the flavor composition in the present invention added to the tobacco product depends on the kind or form of the product, and may be preferably from 0.00001 mass% to 90 mass%, and more preferably from 0.0001 mass% to 50 mass%, based on the mass of the leaf portion of the tobacco.

### Examples

The present invention is described in more detail below by reference to Examples, but is not limited to these examples. In the examples, the terms "part" and "%" mean "mass part" and "mass %" unless otherwise specified.

The analytical instruments used in the examples are as follows.
NMR measurement device: AVANCE III 500 (manufactured by BRUKER BIO SPIN K.K.)
Gas chromatograph-mass spectrometer: GCMS-QP2010 (manufactured by Shimadzu Corporation)
Gas chromatograph-orbitrap mass spectrometer: TRACE1310 (GC) + Exactive GC (mass spectrometer) (manufactured by Thermo Fisher Scientific Inc.)

High performance liquid chromatograph
Pump: LC-20A (manufactured by Shimadzu Corporation)
Detector: SPD-M20A (manufactured by GL Sciences Inc.)
Separation column: YMC-TriartC18 ExRS (manufactured by YMC Co., Ltd.)

### (Example 1) Synthesis of (8Z)-decenal and (8E)-decenal

(8Z)-decenal and (8E)-decenal were synthesized in accordance with the method described in US 8,461,100 B1. That is, 4.63 g of a mixture of 6-decenol, 7-decenol, and 8-decenol was obtained from 5.00 g of 9-decenol, followed by subjecting the mixture to dehydrogenation reaction, thereby obtaining 2.74 g of a mixture of 6-decenol, 7-decenol, and 8-decenal. The mixture was then purified by high-performance liquid chromatography (eluent; water: acetonitrile = 75:25 (volume ratio)), thereby obtaining a desired (8Z)-decenal (GC purity: 100%) and (8E)-decenal (GC purity: 100%).

### <Physical data of (8Z)-decenal>

¹H NMR (500 MHz, (CD₃)₂CO) δ:
9.72 (t, J = 1.72Hz, 1H), 5.34-5.45 (m, 2H), 2.42 (td, J = 7.32, 1.72Hz, 2H), 2.02-2.04 (m, 12H), 1.56-1.63 (m, 5H) 1.28-1.40 (m, 6H)

¹³C NMR (125 MHz, (CD₃)₂CO) δ:
203.47, 132.02, 125.00, 44.93, 30.73, 30.41, 30.37, 27.98, 23.36, 13.48 HRMS (EI⁺): calcd C₁₀H₁₆O(M⁺) 152.1196; found, 152.1196

### <Physical data of (8E)-decenal>

¹H NMR (500 MHz, (CD₃)₂CO) δ:
9.72 (t, J = 1.65Hz, 1H), 5.37-5.45 (m, 2H), 2.42 (td, J = 7.32, 1.72Hz, 2H), 1.92-1.99 (m, 2H), 1.56-1.64 (m, 5H), 1.28-1.37 (m, 6H)

¹³C NMR (125 MHz, (CD₃)₂CO) δ:
203.48, 132.88, 125.97, 44.93, 33.83, 30.81, 30.38, 30.27, 23.35, 18.68 HRMS (EI⁺): calcd C₁₀H₁₆O(M⁺) 152.1196; found, 152.1197

### (Example 2) Grapefruit flavor composition

A grapefruit flavor composition (A) was prepared based on the following formulation using 8-decenal produced by the method described in Example 1.

**Formulation of flavor composition (A)**

| (Ingredients) | (Mass part) |
|---|---|
| (8Z)-decenal | 0.0001 |
| Grapefruit oil (cold pressed) | 50.0 |
| Ethanol | residue |
| Total | 1000.0 |

As a comparison, a grapefruit flavor composition (B) was prepared based on the following formulation.

**Formulation of flavor composition (B)**

| (Ingredients) | (Mass part) |
|---|---|
| Grapefruit oil (cold pressed) | 50.0 |
| Ethanol | residue |
| Total | 1000.0 |

Each of the grapefruit flavor compositions (A) and (B) prepared based on the above formulation was added to water at a concentration of 0.1%, and a sensory test was conducted by seven expert panelists. As a result, all of the panelists commented that the flavor composition (A) was significantly superior to the flavor composition (B) in terms of enhancement of the grapefruit-like peel feeling, juice feeling, and bitter thickness. The same was true for the case where (8Z)-decenal in the formulation of the flavor composition (A) was replaced with (8E)-decenal, and the case where (8Z)-decenal was replaced with an isomeric mixture ((8Z)/(8E) = 90/10, 80/20, 70/30, and 60/40).

The above "(8Z)/(8E)" means "mass of (8Z)-decenal/mass of (8E)-decenal".

### (Example 3) Addition to commercially available grapefruit juice beverage

(8Z)-decenal was added to a commercially available grapefruit juice beverage at a concentration of 0.1 ppb, and a sensory test was conducted by seven expert panelists. As a result, all of the panelists commented that they could feel good flavors because the addition of (8Z)-decenal enhanced the grapefruit-like peel feeling, juice feeling, and bitter thickness. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 4) Grapefruit flavor composition

A grapefruit flavor composition (C) was prepared based on the following formulation using 8-decenal produced by the method described in Example 1.

**Formulation of flavor composition (C)**

| (Ingredients) | (Mass part) |
|---|---|
| (8Z)-decenal | 0.0001 |
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | residue |
| Total | 1000.0 |

As a comparison, a grapefruit flavor composition (D) was prepared based on the following formulation.

**Formulation of flavor composition (D)**

| (Ingredients) | (Mass part) |
|---|---|
| Nootkatone | 2.0 |
| Octanal | 0.3 |
| Decanal | 0.2 |
| Dodecanal | 0.1 |
| Linalool | 0.8 |
| Cis-3-hexenol | 0.5 |
| Ethanol | residue |
| Total | 1000.0 |

Each of the grapefruit flavor compositions (C) and (D) prepared based on the above formulation was added to water at a concentration of 0.1%, and a sensory test was conducted by seven expert panelists. As a result, all of the panelists commented that the flavor composition (C) was significantly superior to the flavor composition (D) in terms of enhancement of the grapefruit-like peel feeling, juice feeling, and bitter thickness. The same was true for the case where (8Z)-decenal in the formulation of the flavor composition (C) was replaced with (8E)-decenal, and the case where (8Z)-decenal was replaced with an isomeric mixture ((8Z)/(8E) = 90/10, 80/20, 70/30, and 60/40).

### (Example 5) Orange flavor composition

An orange flavor composition (E) was prepared based on the following formulation using 8-decenal produced by the method described in Example 1.

**Formulation of flavor composition (E)**

| (Ingredients) | (mass part) |
|---|---|
| (8Z)-decenal | 0.0001 |
| Orange oil (cold pressed) | 50.0 |
| Ethanol | residue |
| Total | 1000.0 |

As a comparison, an orange flavor composition (F) was prepared based on the following formulation.

**Formulation of flavor composition (F)**

| (Ingredients) | (mass part) |
|---|---|
| Orange oil (cold pressed) | 50.0 |
| Ethanol | residue |
| Total | 1000.0 |

Each of the orange flavor compositions (E) and (F) prepared based on the above formulation was added to water at a concentration of 0.1%, and a sensory test was conducted by seven expert panelists. As a result, all of the panelists commented that the flavor composition (E) was significantly superior to the flavor composition (F) in terms of enhancement of the orange-like peel feeling, juice feeling, and bitter thickness. The same was true for the case where (8Z)-decenal in the formulation of the flavor composition (E) was replaced with (8E)-decenal, and the case where (8Z)-decenal was replaced with an isomeric mixture ((8Z)/(8E) = 90/10, 80/20, 70/30, and 60/40).

### (Example 6) Addition to commercially available orange juice beverage

(8Z)-decenal was added to a commercially available orange juice beverage at a concentration of 0.1 ppb, and a sensory test was conducted by seven expert panelists. As a result, all of the panelists commented that they could feel good flavors because the addition of (8Z)-decenal enhanced the orange-like peel feeling, juice feeling, and bitter thickness. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 7) Orange flavor composition

An orange flavor composition (G) was prepared based on the following formulation using 8-decenal produced by the method described in Example 1.

**Formulation of flavor composition (G)**

| (Ingredient) | (Mass part) |
|---|---|
| (8Z)-decenal | 0.0001 |
| Ethyl butyrate | 0.5 |
| Octanal | 1.0 |
| Nonanal | 0.1 |
| Decanal | 0.5 |
| Linalool | 2.0 |
| Ethanol | residue |
| Total | 1000.0 |

As a comparison, an orange flavor composition (H) was prepared based on the following formulation.

**Formulation of flavor composition (H)**

| (Ingredient) | (Mass part) |
|---|---|
| Ethyl butyrate | 0.5 |
| Octanal | 1.0 |
| Nonanal | 0.1 |
| Decanal | 0.5 |
| Linalool | 2.0 |
| Ethanol | residue |
| Total | 1000.0 |

Each of the orange flavor compositions (G) and (H) prepared based on the above formulation was added to water at a concentration of 0.1%, and a sensory test was conducted by seven expert panelists. As a result, all of the panelists commented that the flavor composition (G) was significantly superior to the flavor composition (H) in terms of enhancement of the orange-like peel feeling, juice feeling, and bitter thickness. The same was true for the case where (8Z)-decenal in the formulation of the flavor composition (G) was replaced with (8E)-decenal, and the case where (8Z)-decenal was replaced with an isomeric mixture ((8Z)/(8E) = 90/10, 80/20, 70/30, and 60/40).

### (Example 8) Lemon flavor composition

A lemon flavor composition (I) was prepared based on the following formulation using 8-decenal produced by the method described in Example 1.

**Formulation of flavor composition (I)**

| (Ingredients) | (Mass part) |
|---|---|
| (8Z)-decenal | 0.0001 |
| Lemon oil (cold pressed) | 50.0 |
| Ethanol | residue |
| Total | 1000.0 |

As a comparison, a lemon flavor composition (J) was prepared based on the following formulation.

**Formulation of flavor composition (J)**

| (Ingredients) | (Mass part) |
|---|---|
| Lemon oil (cold pressed) | 50.0 |
| Ethanol | residue |
| Total | 1000.0 |

Each of the lemon flavor compositions (I) and (J) prepared based on the above formulation was added to water at a concentration of 0.1%, and a sensory test was conducted by seven expert panelists. As a result, all of the panelists commented that the flavor composition (I) was significantly superior to the flavor composition (J) in terms of enhancement of the lemon-like peel feeling, juice feeling, and bitter thickness. The same was true for the case where (8Z)-decenal in the formulation of the flavor composition (I) was replaced with (8E)-decenal, and the case where (8Z)-decenal was replaced with an isomeric mixture ((8Z)/(8E) = 90/10, 80/20, 70/30, and 60/40).

### (Example 9) Addition to commercially available lemon juice beverage

(8Z)-decenal was added to a commercially available lemon juice beverage at a concentration of 0.1 ppb, and a sensory test was conducted by seven expert panelists. As a result, all of the panelists commented that they could feel good flavors because the addition of (8Z)-decenal enhanced the lemon-like peel feeling, juice feeling, and bitter thickness. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 10) Lemon flavor composition

A lemon flavor composition (K) was prepared based on the following formulation using 8-decenal produced by the method described in Example 1.

**Formulation of flavor composition (K)**

| (Ingredient) | (Mass part) |
|---|---|
| (8Z)-decenal | 0.0001 |
| Citral | 3.0 |
| α-terpineol | 1.0 |
| Geraniol | 0.5 |
| Geranyl acetate | 0.5 |
| Neryl acetate | 0.5 |
| Ethanol | residue |
| Total | 1000.0 |

As a comparison, a lemon flavor composition (L) was prepared based on the following formulation.

**Formulation of flavor composition (L)**

| (Ingredient) | (Mass part) |
|---|---|
| Citral | 3.0 |
| α-terpineol | 1.0 |
| Geraniol | 0.5 |
| Geranyl acetate | 0.5 |
| Neryl acetate | 0.5 |
| Ethanol | residue |
| Total | 1000.0 |

Each of the lemon flavor compositions (K) and (L) prepared based on the above formulation was added to water at a concentration of 0.1%, and a sensory test was conducted by seven expert panelists. As a result, all of the panelists commented that the flavor composition (K) was significantly superior to the flavor composition (L) in terms of enhancement of the lemon-like peel feeling, juice feeling, and bitter thickness. The same was true for the case where (8Z)-decenal in the formulation of the flavor composition (K) was replaced with (8E)-decenal, and the case where (8Z)-decenal was replaced with an isomeric mixture ((8Z)/(8E) = 90/10, 80/20, 70/30, and 60/40).

### (Example 11) Grapefruit flavor composition for toothpaste

A grapefruit flavor composition for toothpaste (M) was prepared based on the following formulation using 8-decenal produced by the method described in Example 1.

**Formulation of flavor composition (M)**

| (Ingredients) | (Mass part) |
|---|---|
| (8Z)-decenal | 0.0002 |
| L-Menthol | 400.0 |
| Peppermint oil | 150.0 |
| Grapefruit oil (cold pressed) | 100.0 |
| Mint oil | 100.0 |
| Anethole | 60.0 |
| Eucalyptol | 20.0 |
| Ethanol | residue |
| Total | 1000.0 |

As a comparison, a grapefruit flavor composition for toothpaste (N) was prepared based on the following formulation.

**Formulation of flavor composition (N)**

| (Ingredients) | (Mass part) |
|---|---|
| L-Menthol | 400.0 |
| Peppermint oil | 150.0 |
| Grapefruit oil (cold pressed) | 100.0 |
| Mint oil | 100.0 |
| Anethole | 60.0 |
| Eucalyptol | 20.0 |
| Ethanol | residue |
| Total | 1000.0 |

In addition, a toothpaste base material was prepared based on the following formulation.

| (Ingredients) | (Mass part) |
|---|---|
| Calcium carbonate | 400.0 |
| Anhydrous silicic acid | 16.5 |
| Sorbitol (70%) | 240.0 |
| Sodium lauryl sulfate | 13.0 |
| Carboxymethyl cellulose sodium salt | 12.5 |
| Carrageenan | 3.0 |
| Sodium Benzoate | 4.0 |
| Sodium saccharin | 1.5 |
| Purified water | 259.5 |
| Propylene glycol (PG) | 40.0 |
| Total | 990.0 |

The following toothpastes (O) and (P) were prepared using the flavor compositions and toothpaste base material, each prepared in the above formulations, and sensory evaluation was conducted.
Toothpaste (O): Toothpaste base material 990g + flavor composition (M) 10g
Toothpaste (P): Toothpaste base material 990g + flavor composition (N) 10g

A sensory test was conducted for each of these by three expert panelists. As a result, all of the panelists commented that the toothpaste (O) using the flavor composition (M) was significantly superior to the toothpaste (P) using the flavor composition (N) in terms of enhancement of the grapefruit-like peel feeling, juice feeling, and bitter thickness. The same was true for the case where (8Z)-decenal in the formulation of the flavor composition (M) was replaced with (8E)-decenal, and the case where (8Z)-decenal was replaced with an isomeric mixture ((8Z)/(8E) = 90/10, 80/20, 70/30, and 60/40).

### (Example 12) Lime flavor composition

A lime flavor composition (Q) was prepared based on the following formulation using 8-decenal produced by the method described in Example 1.

**Formulation of flavor composition (Q)**

| (Ingredients) | (Mass part) |
|---|---|
| (8Z)-decenal | 0.0001 |
| Lime oil (distilled) | 50.0 |
| Ethanol | residue |
| Total | 1000.0 |

As a comparison, a lime flavor composition (R) was prepared based on the following formulation.

**Formulation of flavor composition (R)**

| (Ingredients) | (Mass part) |
|---|---|
| Lime oil (distilled) | 50.0 |
| Ethanol | residue |
| Total | 1000.0 |

Each of the lime flavor compositions (Q) and (R) prepared based on the above formulation was added to water at a concentration of 0.1%, and a sensory test was conducted by seven expert panelists. As a result, all of the panelists commented that the flavor composition (Q) was significantly superior to the flavor composition (R) in terms of enhancement of the lime-like complexity and peel feeling. The same was true for the case where (8Z)-decenal in the formulation of the flavor composition (Q) was replaced with (8E)-decenal, and the case where (8Z)-decenal was replaced with an isomeric mixture ((8Z)/(8E) = 90/10, 80/20, 70/30, and 60/40).

### (Example 13) Lime flavor composition

A lime flavor composition (S) was prepared based on the following formulation using 8-decenal produced by the method described in Example 1.

**Formulation of flavor composition (S)**

| (Ingredients) | (Mass part) |
|---|---|
| (8Z)-decenal | 0.0001 |
| Citral | 1.0 |
| Terpinen-4-ol | 1.0 |
| 1,8-cineole | 2.0 |
| Geranyl acetate | 1.0 |
| Fenchol | 0.5 |
| Ethanol | residue |
| Total | 1000.0 |

As a comparison, a lime flavor composition (T) was prepared based on the following formulation.

**Formulation of flavor composition (T)**

| (Ingredients) | (Mass part) |
|---|---|
| Citral | 1.0 |
| Terpinen-4-ol | 1.0 |
| 1,8-cineole | 2.0 |
| Geranyl acetate | 1.0 |
| Fenchol | 0.5 |
| Ethanol | residue |
| Total | 1000.0 |

Each of the lime flavor compositions (S) and (T) prepared based on the above formulation was added to water at a concentration of 0.1%, and a sensory test was conducted by seven expert panelists. As a result, all of the panelists commented that the flavor composition (S) was significantly superior to the flavor composition (T) in terms of enhancement of the lime-like complexity and peel feeling. The same was true for the case where (8Z)-decenal in the formulation of the flavor composition (S) was replaced with (8E)-decenal, and the case where (8Z)-decenal was replaced with an isomeric mixture ((8Z)/(8E) = 90/10, 80/20, 70/30, and 60/40).

### (Example 14) Yuzu flavor composition

A yuzu flavor composition (U) was prepared based on the following formulation using 8-decenal produced by the method described in Example 1.

**Formulation of flavor composition (U)**

| (Ingredients) | (Mass part) |
|---|---|
| (8Z)-decenal | 0.0005 |
| Yuzu oil (cold pressed) | 50.0 |
| Ethanol | residue |
| Total | 1000.0 |

As a comparison, a yuzu flavor composition (V) was prepared based on the following formulation.

**Formulation of flavor composition (V)**

| (Ingredients) | (Mass part) |
|---|---|
| Yuzu oil (cold pressed) | 50.0 |
| Ethanol | residue |
| Total | 1000.0 |

Each of the yuzu flavor compositions (U) and (V) prepared based on the above formulation was added to water at a concentration of 0.1%, and a sensory test was conducted by seven expert panelists. As a result, all of the panelists commented that the flavor composition (U) was significantly superior to the flavor composition (V) in terms of enhancement of the yuzu-like peel feeling, juice feeling and complexity. The same was true for the case where (8Z)-decenal in the formulation of the flavor composition (U) was replaced with (8E)-decenal, and the case where (8Z)-decenal was replaced with an isomeric mixture ((8Z)/(8E) = 90/10, 80/20, 70/30, and 60/40).

### (Example 15) Yuzu flavor composition

A yuzu flavor composition (W) was prepared based on the following formulation using 8-decenal produced by the method described in Example 1.

**Formulation of flavor composition (W)**

| (Ingredients) | (Mass part) |
|---|---|
| (8Z)-decenal | 0.0005 |
| Citronellol | 1.0 |
| Octanal | 1.0 |
| Carvacrol | 1.0 |
| Cis-3-hexenal | 1.0 |
| Perillaldehyde | 1.0 |
| Neryl acetate | 2.0 |
| α-terpineol | 2.0 |
| Thymol | 3.0 |
| α-pinene | 8.0 |
| Linalool | 30.0 |
| Myrcene | 50.0 |
| γ-terpinene | 10.0 |
| Limonene | 300.0 |
| Ethanol | residue |
| Total | 1000.0 |

As a comparison, a yuzu flavor composition (X) was prepared based on the following formulation.

**Formulation of flavor composition (X)**

| (Ingredients) | (Mass part) |
|---|---|
| Citronellol | 1.0 |
| Octanal | 1.0 |
| Carvacrol | 1.0 |
| Cis-3-hexenal | 1.0 |
| Perillaldehyde | 1.0 |
| Neryl acetate | 2.0 |
| α-terpineol | 2.0 |
| Thymol | 3.0 |
| α-pinene | 8.0 |
| Linalool | 30.0 |
| Myrcene | 50.0 |
| γ-terpinene | 10.0 |
| Limonene | 300.0 |
| Ethanol | residue |
| Total | 1000.0 |

Each of the yuzu flavor compositions (W) and (X) prepared based on the above formulation was added to water at a concentration of 0.1%, and a sensory test was conducted by seven expert panelists. As a result, all of the panelists commented that the flavor composition (W) was significantly superior to the flavor composition (X) in terms of enhancement of the yuzu-like peel feeling, juice feeling and complexity. The same was true for the case where (8Z)-decenal in the formulation of the flavor composition (W) was replaced with (8E)-decenal, and the case where (8Z)-decenal was replaced with an isomeric mixture ((8Z)/(8E) = 90/10, 80/20, 70/30, and 60/40).

### (Example 16) Soda flavor composition

A soda flavor composition (Y) was prepared based on the following formulation using 8-decenal produced by the method described in Example 1.

**Formulation of flavor composition (Y)**

| (Ingredients) | (Mass part) |
|---|---|
| (8Z)-decenal | 0.00001 |
| Lemon oil (cold pressed) | 10.0 |
| Lime oil (distilled) | 5.0 |
| Orange oil (cold pressed) | 20.0 |
| Isoamyl acetate | 10.0 |
| Ethyl isovalerate | 1.0 |
| Ethyl butyrate | 2.0 |
| Vanillin | 0.005 |
| Ethanol | residue |
| Total | 1000.0 |

As a comparison, a soda flavor composition (Z) was prepared based on the following formulation.

**Formulation of flavor composition (Z)**

| (Ingredients) | (Mass part) |
|---|---|
| Lemon oil (cold pressed) | 10.0 |
| Lime oil (distilled) | 5.0 |
| Orange oil (cold pressed) | 20.0 |
| Isoamyl acetate | 10.0 |
| Ethyl isovalerate | 1.0 |
| Ethyl butyrate | 2.0 |
| Vanillin | 0.005 |
| Ethanol | residue |
| Total | 1000.0 |

Each of the soda flavor compositions (Y) and (Z) prepared based on the above formulation was added to water at a concentration of 0.1%, and a sensory test was conducted by seven expert panelists. As a result, all of the panelists commented that the flavor composition (Y) was significantly superior to the flavor composition (Z) in terms of enhancement of the citrus-like natural volume feeling in soda. The same was true for the case where (8Z)-decenal in the formulation of the flavor composition (Y) was replaced with (8E)-decenal, and the case where (8Z)-decenal was replaced with an isomeric mixture ((8Z)/(8E) = 90/10, 80/20, 70/30, and 60/40).

### (Example 17) Apple flavor composition

An apple flavor composition (1) was prepared based on the following formulation using 8-decenal produced by the method described in Example 1.

**Formulation of flavor composition (1)**

| (Ingredients) | (Mass part) |
|---|---|
| (8Z)-decenal | 0.0001 |
| 2-Methylbutyl acetate | 16.0 |
| Hexanol | 12.0 |
| Trans-2-hexenal | 1.5 |
| Isoamyl alcohol | 10.0 |
| Hexanal | 3.0 |
| Hexyl acetate | 6.0 |
| Butyl acetate | 12.0 |
| Ethyl butyrate | 3.0 |
| Ethyl 2-methylbutyrate | 2.0 |
| Acetic acid | 4.0 |
| Damascenone | 0.005 |
| Ethanol | residue |
| Total | 1000.0 |

As a comparison, an apple flavor composition (2) was prepared based on the following formulation.

**Formulation of flavor composition (2)**

| (Ingredients) | (Mass part) |
|---|---|
| 2-Methylbutyl acetate | 16.0 |
| Hexanol | 12.0 |
| Trans-2-hexenal | 1.5 |
| Isoamyl alcohol | 10.0 |
| Hexanal | 3.0 |
| Hexyl acetate | 6.0 |
| Butyl acetate | 12.0 |
| Ethyl butyrate | 3.0 |
| Ethyl 2-methylbutyrate | 2.0 |
| Acetic acid | 4.0 |
| Damascenone | 0.005 |
| Ethanol | residue |
| Total | 1000.0 |

Each of the apple flavor compositions (1) and (2) prepared based on the above formulation was added to water at a concentration of 0.1%, and a sensory test was conducted by seven expert panelists. As a result, all of the panelists commented that the flavor composition (1) was significantly superior to the flavor composition (2) in terms of impartment of the natural juice feeling. The same was true for the case where (8Z)-decenal in the formulation of the flavor composition (1) was replaced with (8E)-decenal, and the case where (8Z)-decenal was replaced with an isomeric mixture ((8Z)/(8E) = 90/10, 80/20, 70/30, and 60/40).

### (Example 18) Grape flavor composition

A grape flavor composition (3) was prepared based on the following formulation using 8-decenal produced by the method described in Example 1.

**Formulation of flavor composition (3)**

| (Ingredients) | (Mass part) |
|---|---|
| (8Z)-decenal | 0.0001 |
| Ethyl acetate | 30.0 |
| Ethyl butyrate | 20.0 |
| Cis-3-hexenol | 8.0 |
| Methyl anthranilate | 15.0 |
| Ethyl maltol | 20.0 |
| Propionic acid | 15.0 |
| Hexanoic acid | 1.5 |
| Trans-2-hexenal | 2.0 |
| Propyl acetate | 25.0 |
| Ethyl Propionate | 30.0 |
| Linalool | 0.3 |
| Cinnamic alcohol | 0.3 |
| Damascenone | 0.002 |
| Ethanol | residue |
| Total | 1000.0 |

As a comparison, a grape flavor composition (4) was prepared based on the following formulation.

**Formulation of flavor composition (4)**

| (Ingredients) | (Mass part) |
|---|---|
| Ethyl acetate | 30.0 |
| Ethyl butyrate | 20.0 |
| Cis-3-hexenol | 8.0 |
| Methyl anthranilate | 15.0 |
| Ethyl maltol | 20.0 |
| Propionic acid | 15.0 |
| Hexanoic acid | 1.5 |
| Trans-2-hexenal | 2.0 |
| Propyl acetate | 25.0 |
| Ethyl Propionate | 30.0 |
| Linalool | 0.3 |
| Cinnamic alcohol | 0.3 |
| Damascenone | 0.002 |
| Ethanol | residue |
| Total | 1000.0 |

Each of the grape flavor compositions (3) and (4) prepared based on the above formulation was added to water at a concentration of 0.1%, and a sensory test was conducted by seven expert panelists. As a result, all of the panelists commented that the flavor composition (3) was significantly superior to the flavor composition (4) in terms of impartment of the natural juice feeling. The same was true for the case where (8Z)-decenal in the formulation of the flavor composition (3) was replaced with (8E)-decenal, and the case where (8Z)-decenal was replaced with an isomeric mixture ((8Z)/(8E) = 90/10, 80/20, 70/30, and 60/40).

### (Example 19) Peach flavor composition

A peach flavor composition (5) was prepared based on the following formulation using 8-decenal produced by the method described in Example 1.

**Formulation of flavor composition (5)**

| (Ingredients) | (Mass part) |
|---|---|
| (8Z)-decenal | 0.0001 |
| Ethyl acetate | 50.0 |
| Hexanol | 1.0 |
| Benzaldehyde | 0.4 |
| Maltol | 2.0 |
| Hexanal | 0.2 |
| Hexyl acetate | 3.0 |
| γ-undecalactone | 0.3 |
| Ethyl octanoate | 0.05 |
| β-Ionone | 0.002 |
| Linalool | 0.2 |
| Isoamyl acetate | 8.0 |
| Ethyl butyrate | 3.0 |
| Ethanol | residue |
| Total | 1000.0 |

As a comparison, a peach flavor composition (6) was prepared based on the following formulation.

**Formulation of flavor composition (6)**

| (Ingredients) | (Mass part) |
|---|---|
| Ethyl acetate | 50.0 |
| Hexanol | 1.0 |
| Benzaldehyde | 0.4 |
| Maltol | 2.0 |
| Hexanal | 0.2 |
| Hexyl acetate | 3.0 |
| γ-undecalactone | 0.3 |
| Ethyl octanoate | 0.05 |
| β-Ionone | 0.002 |
| Linalool | 0.2 |
| Isoamyl acetate | 8.0 |
| Ethyl butyrate | 3.0 |
| Ethanol | residue |
| Total | 1000.0 |

Each of the peach flavor compositions (5) and (6) prepared based on the above formulation was added to water at a concentration of 0.1%, and a sensory test was conducted by seven expert panelists. As a result, all of the panelists commented that the flavor composition (5) was significantly superior to the flavor composition (6) in terms of impartment of the natural juice feeling. The same was true for the case where (8Z)-decenal in the formulation of the flavor composition (5) was replaced with (8E)-decenal, and the case where (8Z)-decenal was replaced with an isomeric mixture ((8Z)/(8E) = 90/10, 80/20, 70/30, and 60/40).

### (Example 20) Pineapple flavor composition

A pineapple flavor composition (7) was prepared based on the following formulation using 8-decenal produced by the method described in Example 1.

**Formulation of flavor composition (7)**

| (Ingredients) | (Mass part) |
|---|---|
| (8Z)-decenal | 0.0001 |
| Ethyl acetate | 40.0 |
| Allyl hexanoate | 20.0 |
| Ethyl 2-methylbutyrate | 8.0 |
| Ethyl octanoate | 1.0 |
| Ethyl acrylate | 0.02 |
| Ethyl trans-3-hexenoate | 0.04 |
| γ-octalactone | 0.002 |
| 2,5-Dimethyl-4-hydroxy-3(2H)-furanone | 0.3 |
| Ethanol | residue |
| Total | 1000.0 |

As a comparison, a pineapple flavor composition (8) was prepared based on the following formulation.

**Formulation of flavor composition (8)**

| (Ingredients) | (Mass part) |
|---|---|
| Ethyl acetate | 40.0 |
| Allyl hexanoate | 20.0 |
| Ethyl 2-methylbutyrate | 8.0 |
| Ethyl octanoate | 1.0 |
| Ethyl acrylate | 0.02 |
| Ethyl trans-3-hexenoate | 0.04 |
| γ-octalactone | 0.002 |
| 2,5-Dimethyl-4-hydroxy-3(2H)-furanone | 0.3 |
| Ethanol | residue |
| Total | 1000.0 |

Each of the pineapple flavor compositions (7) and (8) prepared based on the above formulation was added to water at a concentration of 0.1%, and a sensory test was conducted by seven expert panelists. As a result, all of the panelists commented that the flavor composition (7) was significantly superior to the flavor composition (8) in terms of impartment of the natural juice feeling. The same was true for the case where (8Z)-decenal in the formulation of the flavor composition (7) was replaced with (8E)-decenal, and the case where (8Z)-decenal was replaced with an isomeric mixture ((8Z)/(8E) = 90/10, 80/20, 70/30, and 60/40).

### (Example 21) Banana flavor composition

A banana flavor composition (9) was prepared based on the following formulation using 8-decenal produced by the method described in Example 1.

**Formulation of flavor composition (9)**

| (Ingredients) | (Mass part) |
|---|---|
| (8Z)-decenal | 0.0001 |
| Isoamyl acetate | 50.0 |
| Isoamyl butyrate | 40.0 |
| Ethyl butyrate | 20.0 |
| Cis-3-hexenol | 5.0 |
| Eugenol | 0.05 |
| Ethanol | residue |
| Total | 1000.0 |

As a comparison, a banana flavor composition (10) was prepared based on the following formulation.

**Formulation of flavor composition (10)**

| (Ingredients) | (Mass part) |
|---|---|
| Isoamyl acetate | 50.0 |
| Isoamyl butyrate | 40.0 |
| Ethyl butyrate | 20.0 |
| Cis-3-hexenol | 5.0 |
| Eugenol | 0.05 |
| Ethanol | residue |
| Total | 1000.0 |

Each of the banana flavor compositions (9) and (10) prepared based on the above formulation was added to water at a concentration of 0.1%, and a sensory test was conducted by seven expert panelists. As a result, all of the panelists commented that the flavor composition (9) was significantly superior to the flavor composition (10) in terms of impartment of the natural juice feeling. The same was true for the case where (8Z)-decenal in the formulation of the flavor composition (9) was replaced with (8E)-decenal, and the case where (8Z)-decenal was replaced with an isomeric mixture ((8Z)/(8E) = 90/10, 80/20, 70/30, and 60/40).

### (Example 22) Strawberry flavor composition

A strawberry flavor composition (11) was prepared based on the following formulation using 8-decenal produced by the method described in Example 1.

**Formulation of flavor composition (11)**

| (Ingredients) | (Mass part) |
|---|---|
| (8Z)-decenal | 0.0001 |
| α-Ionone | 0.1 |
| Ethyl butyrate | 90.0 |
| Ethyl isovalerate | 20.0 |
| Ethyl hexanoate | 1.0 |
| Ethyl 3-methyl-3-phenylglycidate | 10.0 |
| Cis-3-hexenol | 70.0 |
| Nonanoic acid | 3.0 |
| Linalool | 0.4 |
| Ethyl Maltol | 100.0 |
| Vanillin | 0.4 |
| γ-undecalactone | 2.0 |
| γ-dodecalactone | 3.0 |
| Methyl heptyne carbonate | 0.5 |
| Damascenone | 0.002 |
| Propylene glycol (solvent) | residue |
| Total | 1000.0 |

As a comparison, a strawberry flavor composition (12) was prepared based on the following formulation.

**Formulation of flavor composition (12)**

| (Ingredients) | (Mass part) |
|---|---|
| α-Ionone | 0.1 |
| Ethyl butyrate | 90.0 |
| Ethyl isovalerate | 20.0 |
| Ethyl hexanoate | 1.0 |
| Ethyl 3-methyl-3-phenylglycidate | 10.0 |
| Cis-3-hexenol | 70.0 |
| Nonanoic acid | 3.0 |
| Linalool | 0.4 |
| Ethyl Maltol | 100.0 |
| Vanillin | 0.4 |
| γ-undecalactone | 2.0 |
| γ-dodecalactone | 3.0 |
| Methyl heptyne carbonate | 0.5 |
| Damascenone | 0.002 |
| Propylene glycol (solvent) | residue |
| Total | 1000.0 |

Each of the strawberry flavor compositions (11) and (12) prepared based on the above formulation was added to water at a concentration of 0.1%, and a sensory test was conducted by seven expert panelists. As a result, all of the panelists commented that the flavor composition (11) was significantly superior to the flavor composition (12) in terms of impartment of the natural juice feeling. The same was true for the case where (8Z)-decenal in the formulation of the flavor composition (11) was replaced with (8E)-decenal, and the case where (8Z)-decenal was replaced with an isomeric mixture ((8Z)/(8E) = 90/10, 80/20, 70/30, and 60/40).

### (Example 23) Raspberry flavor composition

A raspberry flavor composition (13) was prepared based on the following formulation using 8-decenal produced by the method described in Example 1.

**Formulation of flavor composition (13)**

| (Ingredients) | (Mass part) |
|---|---|
| (8Z)-decenal | 0.0001 |
| α-Ionone | 15.0 |
| Propyl acetate | 200.0 |
| Ethyl butyrate | 90.0 |
| Isoamyl butyrate | 3.0 |
| Trans-2-hexenyl acetate | 30.0 |
| Cis-3-hexenol | 60.0 |
| Trans-2-hexenal | 30.0 |
| Acetic acid | 200.0 |
| 2-Methylbutyrate | 70.0 |
| Linalool | 10.0 |
| γ-decalactone | 5.0 |
| 2-Phenylethyl alcohol | 2.0 |
| Raspberry ketone | 150.0 |
| Damascenone | 10.0 |
| Dimethyl sulfide | 0.2 |
| Propylene glycol (solvent) | residue |
| Total | 1000.0 |

As a comparison, a raspberry flavor composition (14) was prepared based on the following formulation.

**Formulation of flavor composition (14)**

| (Ingredients) | (Mass part) |
|---|---|
| α-Ionone | 15.0 |
| Propyl acetate | 200.0 |
| Ethyl butyrate | 90.0 |
| Isoamyl butyrate | 3.0 |
| Trans-2-hexenyl acetate | 30.0 |
| Cis-3-hexenol | 60.0 |
| Trans-2-hexenal | 30.0 |
| Acetic acid | 200.0 |
| 2-Methylbutyrate | 70.0 |
| Linalool | 10.0 |
| γ-decalactone | 5.0 |
| 2-Phenylethyl alcohol | 2.0 |
| Raspberry ketone | 150.0 |
| Damascenone | 10.0 |
| Dimethyl sulfide | 0.2 |
| Propylene glycol (solvent) | residue |
| Total | 1000.0 |

Each of the raspberry flavor compositions (13) and (14) prepared based on the above formulation was added to water at a concentration of 0.1%, and a sensory test was conducted by seven expert panelists. As a result, all of the panelists commented that the flavor composition (13) was significantly superior to the flavor composition (14) in terms of impartment of the natural juice feeling. The same was true for the case where (8Z)-decenal in the formulation of the flavor composition (13) was replaced with (8E)-decenal, and the case where (8Z)-decenal was replaced with an isomeric mixture ((8Z)/(8E) = 90/10, 80/20, 70/30, and 60/40).

### (Example 24) Blueberry flavor composition

A blueberry flavor composition (15) was prepared based on the following formulation using 8-decenal produced by the method described in Example 1.

**Formulation of flavor composition (15)**

| (Ingredients) | (Mass part) |
|---|---|
| (8Z)-decenal | 0.0001 |
| α-Ionone | 2.0 |
| Isobutyl acetate | 10.0 |
| Isoamyl acetate | 70.0 |
| Ethyl butyrate | 300.0 |
| Dimethylbenzylcarbinyl butyrate | 8.0 |
| Cis-3-hexenyl acetate | 1.0 |
| Cis-3-hexenol | 6.0 |
| Trans-2-hexenal | 3.0 |
| Acetic acid | 300.0 |
| Linalool | 1.0 |
| Geraniol | 0.2 |
| Ethyl Maltol | 10.0 |
| γ-undecalactone | 3.0 |
| 2-Phenylethyl alcohol | 2.0 |
| Raspberry ketone | 100.0 |
| β-Damascones | 1.0 |
| Dimethyl sulfide | 1.0 |
| Propylene glycol (solvent) | residue |
| Total | 1000.0 |

As a comparison, a blueberry flavor composition (16) was prepared based on the following formulation.

**Formulation of flavor composition (16)**

| (Ingredients) | (Mass part) |
|---|---|
| α-Ionone | 2.0 |
| Isobutyl acetate | 10.0 |
| Isoamyl acetate | 70.0 |
| Ethyl butyrate | 300.0 |
| Dimethylbenzylcarbinyl butyrate | 8.0 |
| Cis-3-hexenyl acetate | 1.0 |
| Cis-3-hexenol | 6.0 |
| Trans-2-hexenal | 3.0 |
| Acetic acid | 300.0 |
| Linalool | 1.0 |
| Geraniol | 0.2 |
| Ethyl Maltol | 10.0 |
| γ-undecalactone | 3.0 |
| 2-Phenylethyl alcohol | 2.0 |
| Raspberry ketone | 100.0 |
| β-Damascones | 1.0 |
| Dimethyl sulfide | 1.0 |
| Propylene glycol (solvent) | residue |
| Total | 1000.0 |

Each of the blueberry flavor compositions (15) and (16) prepared based on the above formulation was added to water at a concentration of 0.1%, and a sensory test was conducted by seven expert panelists. As a result, all of the panelists commented that the flavor composition (15) was significantly superior to the flavor composition (16) in terms of impartment of the natural juice feeling. The same was true for the case where (8Z)-decenal in the formulation of the flavor composition (15) was replaced with (8E)-decenal, and the case where (8Z)-decenal was replaced with an isomeric mixture ((8Z)/(8E) = 90/10, 80/20, 70/30, and 60/40).

### (Example 25) Sparkling wine flavor composition

A sparkling wine flavor composition (17) was prepared based on the following formulation using 8-decenal produced by the method described in Example 1.

**Formulation of flavor composition (17)**

| (Ingredients) | (Mass part) |
|---|---|
| (8Z)-decenal | 0.00005 |
| Isoamyl acetate | 3.0 |
| Ethyl hexanoate | 1.0 |
| Ethyl octanoate | 2.0 |
| Ethyl decanoate | 0.6 |
| 2-Phenylethyl alcohol | 1.0 |
| Octanoic acid | 1.0 |
| Decanoic acid | 0.8 |
| Damascenone | 0.005 |
| Ethyl 2-methylbutyrate | 0.05 |
| Ethyl butyrate | 0.3 |
| Geraniol | 0.02 |
| Linalool | 0.03 |
| 2-Phenylethyl acetate | 0.5 |
| Ethyl acetate | 35.0 |
| Isoamyl alcohol | 40.0 |
| Dimethyl sulfide | 0.03 |
| Ethanol | residue |
| Total | 1000.0 |

As a comparison, a sparkling wine flavor composition (18) was prepared based on the following formulation.

**Formulation of flavor composition (18)**

| (Ingredients) | (Mass part) |
|---|---|
| Isoamyl acetate | 3.0 |
| Ethyl hexanoate | 1.0 |
| Ethyl octanoate | 2.0 |
| Ethyl decanoate | 0.6 |
| 2-Phenylethyl alcohol | 1.0 |
| Octanoic acid | 1.0 |
| Decanoic acid | 0.8 |
| Damascenone | 0.005 |
| Ethyl 2-methylbutyrate | 0.05 |
| Ethyl butyrate | 0.3 |
| Geraniol | 0.02 |
| Linalool | 0.03 |
| 2-Phenylethyl acetate | 0.5 |
| Ethyl acetate | 35.0 |
| Isoamyl alcohol | 40.0 |
| Dimethyl sulfide | 0.03 |
| Ethanol | residue |
| Total | 1000.0 |

Each of the sparkling wine flavor compositions (17) and (18) prepared based on the above formulation was added to water at a concentration of 0.1%, and a sensory test was conducted by seven expert panelists. As a result, all of the panelists commented that the flavor composition (17) was significantly superior to the flavor composition (18) in terms of impartment of the ripening feeling and natural volume feeling. The same was true for the case where (8Z)-decenal in the formulation of the flavor composition (17) was replaced with (8E)-decenal, and the case where (8Z)-decenal was replaced with an isomeric mixture ((8Z)/(8E) = 90/10, 80/20, 70/30, and 60/40).

### (Example 26) Beer flavor composition

A beer flavor composition (19) was prepared based on the following formulation using 8-decenal produced by the method described in Example 1.

**Formulation of flavor composition (19)**

| (Ingredients) | (Mass part) |
|---|---|
| (8Z)-decenal | 0.00005 |
| Ethyl acetate | 50.0 |
| Isoamyl alcohol | 50.0 |
| 2-Phenylethyl alcohol | 20.0 |
| Octanoic acid | 8.0 |
| Hexanoic acid | 3.0 |
| Decanoic acid | 0.5 |
| Isoamyl acetate | 2.0 |
| 2-Phenylethyl acetate | 2.0 |
| Methionol | 1.0 |
| Ethyl octanoate | 0.5 |
| Ethyl hexanoate | 0.1 |
| Ethyl decanoate | 0.05 |
| 4-Vinylguaiacol | 0.2 |
| γ-nonalactone | 0.02 |
| Linalool | 0.01 |
| Sotolon | 0.002 |
| Damascenone | 0.001 |
| Propylene glycol (solvent) | residue |
| Total | 1000.0 |

As a comparison, a beer flavor composition (20) was prepared based on the following formulation.

**Formulation of flavor composition (20)**

| (Ingredients) | (Mass part) |
|---|---|
| Ethyl acetate | 50.0 |
| Isoamyl alcohol | 50.0 |
| 2-Phenylethyl alcohol | 20.0 |
| Octanoic acid | 8.0 |
| Hexanoic acid | 3.0 |
| Decanoic acid | 0.5 |
| Isoamyl acetate | 2.0 |
| 2-Phenylethyl acetate | 2.0 |
| Methionol | 1.0 |
| Ethyl octanoate | 0.5 |
| Ethyl hexanoate | 0.1 |
| Ethyl decanoate | 0.05 |
| 4-Vinylguaiacol | 0.2 |
| γ-nonalactone | 0.02 |
| Linalool | 0.01 |
| Sotolon | 0.002 |
| Damascenone | 0.001 |
| Propylene glycol (solvent) | residue |
| Total | 1000.0 |

Each of the beer flavor compositions (19) and (20) prepared based on the above formulation was added to water at a concentration of 0.1%, and a sensory test was conducted by seven expert panelists. As a result, all of the panelists commented that the flavor composition (19) was significantly superior to the flavor composition (20) in terms of impartment of the grain feeling and natural volume feeling. The same was true for the case where (8Z)-decenal in the formulation of the flavor composition (19) was replaced with (8E)-decenal, and the case where (8Z)-decenal was replaced with an isomeric mixture ((8Z)/(8E) = 90/10, 80/20, 70/30, and 60/40).

### (Example 27) Green Tea Flavor Composition

A green tea flavor composition (21) was prepared based on the following formulation using 8-decenal produced by the method described in Example 1.

**Formulation of flavor composition (21)**

| (Ingredients) | (Mass part) |
|---|---|
| (8Z)-decenal | 0.00001 |
| α-Ionone | 0.1 |
| Cis-3-hexenyl hexanoate | 5.0 |
| Methyl jasmonate | 3.0 |
| Hexanal | 0.3 |
| Hexanol | 2.0 |
| Cis-3-hexenol | 1.0 |
| α-terpineol | 10.0 |
| Linalool | 1.0 |
| Nerol | 2.0 |
| Phenylacetaldehyde | 2.0 |
| Cis-Jasmone | 8.0 |
| β-Ionone | 1.0 |
| Indole | 0.6 |
| Dimethyl sulfide | 5.0 |
| Propylene glycol (solvent) | residue |
| Total | 1000.0 |

As a comparison, a green tea flavor composition (22) was prepared based on the following formulation.

**Formulation of flavor composition (22)**

| (Ingredients) | (Mass part) |
|---|---|
| α- Ionone | 0.1 |
| Cis-3-hexenyl hexanoate | 5.0 |
| Methyl jasmonate | 3.0 |
| Hexanal | 0.3 |
| Hexanol | 2.0 |
| Cis-3-hexenol | 1.0 |
| α-terpineol | 10.0 |
| Linalool | 1.0 |
| Nerol | 2.0 |
| Phenylacetaldehyde | 2.0 |
| Cis-Jasmone | 8.0 |
| β-Ionone | 1.0 |
| Indole | 0.6 |
| Dimethyl sulfide | 5.0 |
| Propylene glycol (solvent) | residue |
| Total | 1000.0 |

Each of the green tea flavor compositions (21) and (22) prepared based on the above formulation was added to water at a concentration of 0.1%, and a sensory test was conducted by seven expert panelists. As a result, all of the panelists commented that the flavor composition (21) was significantly superior to the flavor composition (22) in terms of impartment of the natural tea leaf feeling. The same was true for the case where (8Z)-decenal in the formulation of the flavor composition (21) was replaced with (8E)-decenal, and the case where (8Z)-decenal was replaced with an isomeric mixture ((8Z)/(8E) = 90/10, 80/20, 70/30, and 60/40).

### (Example 28) Black tea flavor composition

A black tea flavor composition (23) was prepared based on the following formulation using 8-decenal produced by the method described in Example 1.

Formulation of flavor composition (23)

| (Ingredients) | (Mass part) |
|---|---|
| (8Z)-decenal | 0.00001 |
| 2-Phenylethyl alcohol | 1.0 |
| Geraniol | 1.0 |
| Linalool oxide | 0.5 |
| Hexanal | 0.05 |
| Phenylacetaldehyde | 0.1 |
| Cis-3-hexenol | 1.0 |
| β-Ionone | 0.001 |
| Linalool | 1.0 |
| Damascenone | 0.03 |
| Isoeugenol | 0.001 |
| Propylene glycol (solvent) | residue |
| Total | 1000.0 |

As a comparison, a black tea flavor composition (24) was prepared based on the following formulation.

**Formulation of flavor composition (24)**

| (Ingredients) | (Mass part) |
|---|---|
| 2-Phenylethyl alcohol | 1.0 |
| Geraniol | 1.0 |
| Linalool oxide | 0.5 |
| Hexanal | 0.05 |
| Phenylacetaldehyde | 0.1 |
| Cis-3-hexenol | 1.0 |
| β-Ionone | 0.001 |
| Linalool | 1.0 |
| Damascenone | 0.03 |
| Isoeugenol | 0.001 |
| Propylene glycol (solvent) | residue |
| Total | 1000.0 |

Each of the black tea flavor compositions (23) and (24) prepared based on the above formulation was added to water at a concentration of 0.1%, and a sensory test was conducted by seven expert panelists. As a result, all of the panelists commented that the flavor composition (23) was significantly superior to the flavor composition (24) in terms of impartment of the natural tea leaf feeling. The same was true for the case where (8Z)-decenal in the formulation of the flavor composition (23) was replaced with (8E)-decenal, and the case where (8Z)-decenal was replaced with an isomeric mixture ((8Z)/(8E) = 90/10, 80/20, 70/30, and 60/40).

### (Example 29) Addition to commercially available lime juice beverage

(8Z)-decenal was added to a commercially available lime juice beverage at a concentration of 0.1 ppb, and a sensory test was conducted by seven expert panelists. As a result, all of the panelists commented that they could feel good flavors because the addition of (8Z)-decenal enhanced the lime-like complexity and peel feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 30) Addition to commercially available yuzu juice beverage

(8Z)-decenal was added to a commercially available yuzu juice beverage at a concentration of 0.5 ppb, and a sensory test was conducted by seven expert panelists. As a result, all of the panelists commented that they could feel good flavors because the addition of (8Z)-decenal enhanced the yuzu-like peel feeling, juice feeling and complexity. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 31) Addition to commercially available soda beverage

(8Z)-decenal was added to a commercially available soda beverage at a concentration of 0.01 ppb, and a sensory test was conducted by seven expert panelists. As a result, all of the panelists commented that they could feel good flavors because the addition of (8Z)-decenal enhanced the citrus-like natural volume feeling in soda. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 32) Addition to commercially available apple juice beverage

(8Z)-decenal was added to a commercially available apple juice beverage at a concentration of 0.1 ppb, and a sensory test was conducted by seven expert panelists. As a result, all of the panelists commented that they could feel good flavors because the addition of (8Z)-decenal imparted the natural juice feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 33) Addition to commercially available grape juice beverage

(8Z)-decenal was added to a commercially available grape juice beverage at a concentration of 0.1 ppb, and a sensory test was conducted by seven expert panelists. As a result, all of the panelists commented that they could feel good flavors because the addition of (8Z)-decenal imparted the natural juice feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 34) Addition to commercially available peach juice beverage

(8Z)-decenal was added to a commercially available peach juice beverage at a concentration of 0.1 ppb, and a sensory test was conducted by seven expert panelists. As a result, all of the panelists commented that they could feel good flavors because the addition of (8Z)-decenal imparted the natural juice feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 35) Addition to commercially available sparkling wine taste beverage

(8Z)-decenal was added to a commercially available sparkling wine taste beverage at a concentration of 0.05 ppb, and a sensory test was conducted by seven expert panelists. As a result, all of the panelists commented that they could feel good flavors because the addition of (8Z)-decenal imparted the ripening feeling and natural volume feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 36) Addition to commercially available beer taste beverage

(8Z)-decenal was added to a commercially available beer taste beverage at a concentration of 0.05 ppb, and a sensory test was conducted by seven expert panelists. As a result, all of the panelists commented that they could feel good flavors because the addition of (8Z)-decenal imparted the grain feeling and natural volume feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 37) Addition to commercially available green tea beverage

(8Z)-decenal was added to a commercially available green tea beverage at a concentration of 0.01 ppb, and a sensory test was conducted by seven expert panelists. As a result, all of the panelists commented that they could feel good flavors because the addition of (8Z)-decenal imparted the natural tea leaf feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

### (Example 38) Addition to commercially available black tea beverage

(8Z)-decenal was added to a commercially available black tea beverage at a concentration of 0.01 ppb, and a sensory test was conducted by seven expert panelists. As a result, all of the panelists commented that they could feel good flavors because the addition of (8Z)-decenal imparted the natural tea leaf feeling. The same was true for the case where (8Z)-decenal was replaced with (8E)-decenal, and the case where (8Z)-decenal was replaced with an isomeric mixture ((8Z)/(8E) = 90/10, 80/20, 70/30, and 60/40).

Although the present invention is described in detail and with reference to specific embodiments, it is obvious to those skilled in the art that various changes and modifications can be made without departing from the spirit and scope of the present invention. This application is based on Japanese patent application No. 2018-239524 filed on December 21, 2018 and Japanese patent application No. 2019-152072 filed on August 22, 2019, the contents of which are incorporated herein by reference.

## Claims

1. A flavor composition comprising 8-decenal.

2. The flavor composition according to claim 1, wherein 8-decenal is (8Z)-decenal.

3. The flavor composition according to claim 1 or 2, which is for addition to at least one product selected from the group consisting of foods or beverages, oral compositions, and tobacco products.

4. The flavor composition according to claim 3, wherein the product has a citrus-like flavor.

5. The flavor composition according to claim 4, wherein the citrus is at least one selected from the group consisting of grapefruit, orange, lemon, lime, yuzu, blood orange, bitter orange, tangerine, mandarin orange, satsuma mandarin orange, amanatsu, natsumikan, hassaku, pomelo, kabosu, sudachi, hebesu, bergamot, and sweetie (oroblanco).

6. A food or beverage, oral composition or tobacco product, comprising the flavor composition according to any one of claims 1 to 5.

7. A method for producing a food or beverage, oral composition, or tobacco product, the method comprising adding the flavor composition according to any one of claims 1 to 5.
